# EUROPÄISCHE PATENTANMELDUNG

(11) **EP 4 016 063 A1**
(43) Veröffentlichungstag der Anmeldung: **22.06.2022**
(21) Anmeldenummer: 21214005.7
(22) Anmeldetag: 13.12.2021
(51) Int. Cl.: G01N 24/08, G01R 33/46, G06N 3/04, G16C 20/20, G16C 20/70, G06N 3/08

(54) **ERZEUGEN VON CODES FÜR CHEMISCHE STRUKTUREN AUS NMR-SPEKTROSKOPISCHEN DATEN**

(30) Priorität: 16.12.2020 EP 20214534
(71) Anmelder: Bayer Aktiengesellschaft, 51373 Leverkusen (DE)
(72) Erfinder: Clevert, Djork-Arne, 10437 Berlin (DE); Winter, Jan, Robin, 10115 Berlin (DE)
(74) Vertreter: BIP Patents

(57) **Zusammenfassung**

Die vorliegende Erfindung befasst sich mit der Strukturaufklärung chemischer Verbindungen. Gegenstände der vorliegenden Erfindung sind ein Verfahren, ein Computersystem und ein Computerprogrammprodukt zur Erzeugung von Strukturcodes für chemische Verbindungen aus spektroskopischen Daten der chemischen Verbindungen.

## Beschreibung

Die vorliegende Erfindung befasst sich mit der Strukturaufklärung chemischer Verbindungen. Gegenstände der vorliegenden Erfindung sind ein Verfahren, ein Computersystem und ein Computerprogrammprodukt zum Erzeugen von Strukturcodes für chemische Verbindungen aus spektroskopischen Daten der chemischen Verbindungen.

Die chemische Struktur gibt den Aufbau auf molekularer oder ionischer Ebene eines einheitlichen (homogenen) Stoffes wieder. Sie gibt an, wie Atome, Atomgruppen, Ionen, Bindungen und/oder freie Elektronenpaare zueinander angeordnet sind.

Zur Aufklärung der Struktur chemischer Verbindungen gibt es verschiedene Verfahren. Zur Bestimmung der Struktur in Flüssigkeiten, Gasen und amorphen Festkörpern eignen sich insbesondere die folgenden Methoden: NMR-Spektroskopie, Massenspektrometrie, Schwingungsspektroskopie, UV-Vis-Spektroskopie und Röntgenabsorptionsspektroskopie.

Die NMR-Spektroskopie (NMR: *nuclear magnetic resonance;* deutsch: Kernspinresonanzspektroskopie) ist eine spektroskopische Methode zur Untersuchung der elektronischen Umgebung einzelner Atome und der Wechselwirkungen mit den Nachbaratomen. Die Methode beruht auf der magnetischen Kernresonanz, einer resonanten Wechselwirkung zwischen dem magnetischen Moment von Atomkernen der Probe, die sich in einem statischen Magnetfeld befindet, mit einem hochfrequenten magnetischen Wechselfeld. Es sind nur solche Isotope der Spektroskopie zugänglich, die im Grundzustand einen von Null verschiedenen Kernspin und damit ein magnetisches Moment besitzen, zum Beispiel ¹H, ²D, ⁶Li, ¹⁰B, ¹¹B, ¹³C, ¹⁵N, ¹⁷O, ¹⁹F, ³¹P und ⁴³Ca.

Die ¹³C-NMR-Spektroskopie ist in Kombination mit der ¹H-NMR-Spektroskopie die wichtigste Methode für die Strukturaufklärung organischer Moleküle. Anhand eines ¹³C-Spektrums und eines ¹H-NMR-Spektrums einer chemischen Verbindung ist ein Chemiker üblicherweise in der Lage, die Struktur der chemischen Verbindung zu entschlüsseln und damit die chemische Verbindung zu bestimmen / zu identifizieren. Zur Strukturaufklärung organischer chemischer Verbindungen sind eine Vielzahl an Fachbüchern erschienen (siehe z.B. H. Duddeck, W. Dietrich: Strukturaufklärung mit moderner NMR-Spektroskopie, Steinkopf-Verlag, 1988, ISBN: 978-3-642-97777-0; E. Pretsch et al.: Spektroskopische Daten zur Strukturaufklärung organischer Verbindungen, 4. Auflage, Springer-Verlag, 2001, ISBN: 978-3-540-41877-1).

Da die Strukturaufklärung anhand spektroskopischer Daten eine mitunter zeitraubende Angelegenheit sein kann, wäre es wünschenswert, diese beschleunigen zu können.

Dies wird durch die vorliegende Erfindung erreicht.

Ein erster Gegenstand der vorliegenden Erfindung ist ein computer-implementiertes Verfahren umfassend die Schritte:
- Empfangen von spektroskopischen Daten einer chemischen Verbindung,
- Zuführen der spektroskopischen Daten einem ersten künstlichen neuronalen Netz, wobei das erste künstliche neuronale Netz in einem überwachten Lernverfahren anhand von spektroskopischen Daten einer Vielzahl von chemischen Referenzverbindungen trainiert worden ist, auf Basis der spektroskopischen Daten der chemischen Referenzverbindungen molekulare Deskriptoren der chemischen Referenzverbindungen zu erzeugen,
- Empfangen eines molekularen Deskriptors der chemischen Verbindung von dem ersten künstlichen neuronalen Netz,
- Zuführen des empfangenen molekularen Deskriptors einem zweiten künstlichen neuronalen Netz, wobei das zweite künstliche neuronale Netz ein Decoder eines Autoencoders ist, wobei der Autoencoder in einem unüberwachten Lernverfahren anhand einer Vielzahl von chemischen Referenzverbindungen trainiert worden ist, Strukturdaten der chemischen Referenzverbindungen in molekulare Deskriptoren zu überführen und die molekularen Deskriptoren wieder in die Strukturdaten zu überführen,
- Empfangen von Strukturdaten der chemischen Verbindung von dem zweiten künstlichen neuronalen Netz,
- Ausgeben und/oder Speichern der Strukturdaten und/oder von Informationen, die von den Strukturdaten abgeleitet sind.

Ein weiterer Gegenstand der vorliegenden Erfindung ist ein Computersystem umfassend
- eine Eingabeeinheit,
- eine Steuer- und Recheneinheit und
- eine Ausgabeeinheit
   - wobei die Steuer- und Recheneinheit konfiguriert ist, die Eingabeeinheit zu veranlassen, spektroskopische Daten einer chemischen Verbindung zu empfangen,
   - wobei die Steuer- und Recheneinheit konfiguriert ist, die spektroskopischen Daten einem ersten künstlichen neuronalen Netz zuzuführen, wobei das erste künstliche neuronale Netz in einem überwachten Lernverfahren anhand von spektroskopischen Daten einer Vielzahl von chemischen Referenzverbindungen trainiert worden ist, auf Basis der spektroskopischen Daten der chemischen Referenzverbindungen molekulare Deskriptoren der chemischen Referenzverbindungen zu erzeugen,
   - wobei die Steuer- und Recheneinheit konfiguriert ist, von dem ersten künstlichen neuronalen Netz einen molekularen Deskriptor für die chemische Verbindung zu empfangen,
   - wobei die Steuer- und Recheneinheit konfiguriert ist, den empfangenen molekularen Deskriptor einem zweiten künstlichen neuronalen Netz zuzuführen, wobei das zweite künstliche neuronale Netz ein Decoder eines Autoencoders ist, wobei der Autoencoder in einem unüberwachten Lernverfahren anhand einer Vielzahl von chemischen Referenzverbindungen trainiert worden ist, Strukturdaten der chemischen Referenzverbindungen in molekulare Deskriptoren zu überführen und die molekularen Deskriptoren wieder in die Strukturdaten zu überführen,
   - wobei die Steuer- und Recheneinheit konfiguriert ist, von dem zweiten künstlichen neuronalen Netz Strukturdaten der chemischen Verbindung zu empfangen,
   - wobei die Steuer- und Recheneinheit konfiguriert ist, die Ausgabeeinheit zu veranlassen, die empfangenen Strukturdaten und/oder Informationen, die von den Strukturdaten abgeleitet sind, auszugeben und/oder zu speichern.

Ein weiterer Gegenstand der vorliegenden Erfindung ist ein Computerprogrammprodukt umfassend ein Computerprogramm, das in einen Arbeitsspeicher eines Computersystems geladen werden kann und dort das Computersystem dazu veranlasst, folgende Schritte ausführen:
- Empfangen von spektroskopischen Daten einer chemischen Verbindung,
- Zuführen der spektroskopischen Daten einem ersten künstlichen neuronalen Netz, wobei das erste künstliche neuronale Netz in einem überwachten Lernverfahren anhand von spektroskopischen Daten einer Vielzahl von chemischen Referenzverbindungen trainiert worden ist, auf Basis der spektroskopischen Daten der chemischen Referenzverbindungen molekulare Deskriptoren der chemischen Referenzverbindungen zu erzeugen,

- Empfangen eines molekularen Deskriptors der chemischen Verbindung von dem ersten künstlichen neuronalen Netz,
- Zuführen des empfangenen molekularen Deskriptors einem zweiten künstlichen neuronalen Netz, wobei das zweite künstliche neuronale Netz ein Decoder eines Autoencoders ist, wobei der Autoencoder in einem unüberwachten Lernverfahren anhand einer Vielzahl von chemischen Referenzverbindungen trainiert worden ist, Strukturdaten der chemischen Referenzverbindungen in molekulare Deskriptoren zu überführen und die molekularen Deskriptoren wieder in die Strukturdaten zu überführen,
- Empfangen von Strukturdaten der chemischen Verbindung von dem zweiten künstlichen neuronalen Netz,
- Ausgeben und/oder Speichern der Strukturdaten und/oder von Informationen, die von den Strukturdaten abgeleitet sind.

Die Erfindung wird nachstehend näher erläutert, ohne zwischen den Erfindungsgegenständen (Verfahren, Computersystem, Computerprogrammprodukt) zu unterscheiden. Die nachfolgenden Erläuterungen sollen vielmehr für alle Erfindungsgegenstände in analoger Weise gelten, unabhängig davon, in welchem Kontext (Verfahren, Computersystem, Computerprogrammprodukt) sie erfolgen.

Die vorliegende Erfindung erzeugt aus spektroskopischen Daten einer chemischen Verbindung Strukturdaten für die chemische Verbindung.

Unter dem Begriff "chemische Verbindung" wird ein Reinstoff verstanden, der aus Atomen von zwei oder mehreren chemischen Elementen besteht, wobei (im Gegensatz zu Gemischen) die Atomarten zueinander in einem festen Verhältnis stehen. Das zahlenmäßige Verhältnis der Atome zueinander wird durch chemische Bindungen zwischen den Atomen bestimmt, und das Verhältnis lässt sich in einer Summenformel darstellen.

Vorzugsweise handelt es sich bei der chemischen Verbindung um eine organische Verbindung. Eine "organische Verbindung" ist eine chemische Verbindung, die Kohlenstoff-Wasserstoff-Bindungen (C-H-Bindungen) umfasst. Vorzugsweise handelt es sich um eine organische Verbindung, deren Moleküle lediglich aus den folgenden Elementen aufgebaut sind: Kohlenstoff (C), Wasserstoff (H), Sauerstoff (O), Stickstoff (N), Schwefel (S), Fluor (F), Chlor (Cl), Brom (Br), Iod (I) und/oder Phosphor (P).

Unter dem Begriff "Strukturdaten" werden Informationen verstanden, aus denen sich die chemische Struktur der chemischen Verbindung ableiten lässt. Dabei definiert die chemische Struktur den Aufbau der chemischen Verbindung auf molekularer oder ionischer Ebene. Mit anderen Worten: die chemische Struktur gibt an, wie Atome, Atomgruppen, Ionen und Bindungen bzw. freie Elektronenpaare zueinander angeordnet sind.

Vorzugsweise handelt es sich bei den Strukturdaten um Informationen, aus denen sich die komplette chemische Struktur einer chemischen Verbindung herauslesen lässt. Beispiele solcher Strukturdaten sind Strukturcodes, in denen die chemische Struktur in Form einer maschinell verarbeitbaren Zeichenkette kodiert ist. Eine solche Zeichenkette umfasst Zeichen eines definierten Zeichensatzes, d.h. eines definierten Vorrats an Elementen. Solche Elemente (Zeichen) können unter anderem die Buchstaben eines Alphabetes, Ziffern, aber auch andere Symbole sein, wie beispielsweise Sonderzeichen, Satzzeichen, die Zeichen der Lautumschrift des IPA-Codes (IPA: Internationales Phonetisches Alphabet) und/oder der Brailleschrift, Piktogramme verschiedener Art und/oder Steuerzeichen. Beispiele für bekannte Zeichensätze sind ASCII (*American Standard Code for Information Interchange*), EBCDIC (*Extended Binary Coded Decimal Interchange Code*) und Unicode. Die Abbildung von einer chemischen Struktur einer chemischen Verbindung auf einen Strukturcode ist üblicherweise eindeutig (für jede chemische Struktur gibt es genau einen Strukturcode) und reversibel (aus dem Strukturcode lässt sich die chemische Struktur rekonstruieren, z.B. in Form einer bildhaften Darstellung).

Beispiele für solche Strukturcodes sind SMILES-Codes, InChI-Codes, CML-Codes und WLN-Codes. SMILES steht für *Simplified Molecular Input Line Entry Specification.* Dabei handelt es sich um einen chemischen Strukturcode, bei dem die Struktur beliebiger Moleküle stark vereinfacht als ASCII-Zeichenkette wiedergegeben wird. Mehrere kommerziell verfügbare Molekül-Editoren können SMILES-Strings importieren und so zweidimensionale und dreidimensionale Modelle der Moleküle erzeugen. Auch der *IUPAC International Chemical Identifier* (InChI) ist ein chemischer Strukturcode, der es ermöglicht, ein Molekül eindeutig und reversibel in eine standardisierte Zeichenkette zu übersetzen. Ein weiteres Beispiel ist die *Chemical Markup Language* (ChemML oder CML), ein auf XML (*Extensible Markup Language*) basierendes Datenformat, das unter anderem zur Darstellung von chemischen Formeln verwendet werden kann. Ein weiteres Beispiel ist die *Wiswesser Line Notation* (WLN).

Auch bei dem IUPAC-Namen einer chemischen Verbindung handelt es sich um Strukturdaten im Sinne der vorliegenden Erfindung.

Unter "spektroskopischen Daten" werden Informationen verstanden, die das Ergebnis eines spektroskopischen und/oder spektrometrischen Messverfahrens an der chemischen Verbindung sind. Bei spektroskopischen Messverfahren kommt es zu einer Wechselwirkung zwischen der chemischen Verbindung und elektromagnetischer Strahlung. Dabei wird die elektromagnetische Strahlung nach einer bestimmten Eigenschaft wie Wellenlänge und/oder Energie zerlegt und die Wechselwirkung zwischen der chemischen Verbindung und der elektromagnetischen Strahlung analysiert. Das Ergebnis dieser Analyse ist üblicherweise ein Spektrum. Vorzugsweise handelt es sich bei den spektroskopischen Daten um ein oder mehrere NMR-Spektren, Infrarotspektren und/oder UV-Vis-Spektren. Messverfahren, die nicht auf der Wechselwirkung der chemischen Verbindung mit elektromagnetischer Strahlung beruhen, werden in dieser Beschreibung als spektrometrische Messverfahren bezeichnet; ein bekanntes Beispiel ist die Massenspektrometrie. Eine Übersicht gängiger spektroskopischer und spektrometrischer Messverfahren gibt beispielsweise das folgende Werk: Encyclopedia of Spectroscopy and Spectrometry, J. C. Lindon, G. E. Tranter, D. W. Koppenaal, Elsevier 2017, Bände 1, 2, 3 und 4, ISBN 9780128105382, 9780128105368, 9780128105375, 9780128105511.

In einer besonders bevorzugten Form handelt es sich bei den spektroskopischen Daten um eine oder mehrere Peak-Listen eines Spektrums oder mehrerer Spektren der chemischen Verbindung, besonders bevorzugt um mindestens eine Peak-Liste eines ¹H-NMR- und/oder um mindestens eine Peak-Liste eines ¹³C-NMR- Spektrums.

Ein (NMR-)Spektrum einer chemischen Verbindung enthält eine Reihe lokaler Extrema (Maxima und Minima), die allgemein als Peaks bezeichnet werden. Diese lokalen Extrema können durch Parameter wie Lage auf der Abszisse (im Fall von NMR-Spektren Frequenz oder chemische Verschiebung), Intensität, Linienbreite und/oder Volumen charakterisiert werden. Um lokale Extrema in (NMR-)Spektren zu analysieren und damit zu arbeiten, werden die Peaks in dem Spektrum identifiziert, ihre Parameter erfasst und in Form der Peak-Liste als Deskriptor gespeichert. Für die automatisierte Analyse von Spektren, die Identifizierung von Peaks, die Bestimmung der Peak-Parameter und die Erzeugung einer Peak-Liste sind kommerzielle und frei verfügbare Softwarewerkzeuge vorhanden (z.B. TopSpin, Mnova, INFOS, NMRPipe u.a.).

Ganz besonders bevorzugt umfassen die spektroskopischen Daten der chemischen Verbindung eine Peak-Liste eines ¹³C-NMR-Spektrums der chemischen Verbindung, wobei in der Peak-Liste alle lokalen Extrema in dem ¹³C-NMR-Spektrum anhand der folgenden Parameter spezifiziert sind: Frequenz oder chemische Verschiebung, Intensität, Linienbreite und/oder Peak-Integral).

Bei dem ¹³C-NMR-Spektrum / den ¹³C-NMR-Spektren der chemischen Verbindung kann es sich um ein ¹H-Breitband-entkoppeltes Spektrum, um ein DEPT-Spektrum (z.B. ein DEPT-135- oder ein DEPT-90-Spektrum), um ein APT-Spektrum, um ein ¹H-Off-Resonance-Spektrum und/oder um ein Gated-Decoupling-Spektrum handeln.

Details zu den genannten Spektren und weiteren Spektren sowie zur Aufnahme von NMR-Spektren sind den zahlreichen Fachbüchern zu diesem Thema zu entnehmen (siehe z.B. C. Schorn, B. J. Taylor: NMR-Spectroscopy: Data Acquisition, 2. Auflage, Wiley-Verlag 2005, ISBN: 978-3-527-60619-1; T.N. Mitchell, B. Costisella: NMR - From Spectra to Structures, Springer-Verlag 2013, ISBN: 9783662054345).

Die Erzeugung von Strukturdaten einer chemischen Verbindung anhand von spektroskopischen Daten kann in mehreren Schritten erfolgen; in einem ersten Schritt wird anhand der spektroskopischen Daten mit Hilfe eines ersten künstlichen neuronalen Netzes ein molekularer Deskriptor erzeugt und in einem zweiten Schritt werden aus dem molekularen Deskriptor mit Hilfe eines zweiten künstlichen neuronalen Netzes Strukturdaten erzeugt.

Der molekulare Deskriptor ist, wie die Strukturdaten und insbesondere der chemische Strukturcode auch, eine eineindeutige Repräsentation der chemischen Struktur der chemischen Verbindung: aus einer chemischen Struktur (repräsentiert durch die Strukturdaten) lässt sich genau ein molekularer Deskriptor erzeugen und aus dem molekularen Deskriptor lässt sich genau eine chemische Struktur (repräsentiert durch die Strukturdaten) erzeugen. Während die Strukturdaten, insbesondere der chemische Strukturcode üblicherweise eine variable Länge (der Zeichenkette) aufweist, hat der molekulare Deskriptor eine feste Größe, d.h. eine feste Anzahl an Bits/Bytes zur digitalen Darstellung, unabhängig von der Komplexität der chemischen Struktur. Der molekulare Deskriptor kann beispielsweise ein n-dimensionaler Vektor sein, wobei n eine ganze Zahl ist, wobei n beispielsweise die Werte 128, 256, 512, 1024 oder andere Werte annehmen kann. Details zum molekularen Deskriptor und zu seiner Erzeugung sind weiter unten in der Beschreibung zu finden.

In einem ersten Schritt werden spektroskopische Daten einer chemischen Verbindung empfangen. In einem weiteren Schritt werden die spektroskopischen Daten dem ersten künstlichen neuronalen Netz zugeführt

Ein solches künstliches neuronales Netzwerk umfasst mindestens drei Schichten von Verarbeitungselementen: eine erste Schicht mit Eingangsneuronen (Knoten), eine N-te Schicht mit mindestens einem Ausgangsneuron (Knoten) und N-2 innere Schichten, wobei N eine natürliche Zahl und größer als 2 ist.

Die Eingangsneuronen des ersten künstlichen neuronalen Netzes dienen zum Empfangen der spektroskopischen Daten.

Die Ausgangsneuronen des ersten künstlichen neuronalen Netzes dienen zur Ausgabe des molekularen Deskriptors.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Das künstliche neuronale Netz ist konfiguriert, aus spektroskopischen Daten einer chemischen Verbindung einen molekularen Deskriptor als Ausgabe zu erzeugen. Die Konfiguration des neuronalen Netzes erfolgt üblicherweise mittels eines überwachten Lernverfahrens (engl.: *supervised learning*).

Das Lernen/Trainieren erfolgt mit Hilfe von Trainingsdaten einer Vielzahl von chemischen Referenzverbindungen. Die Trainingsdaten umfassen für jede chemische Referenzverbindung der Vielzahl an chemischen Referenzverbindungen spektroskopische Daten (Eingabevektor) und einen molekularen Deskriptor (Ausgabevektor).

Das Trainieren des neuronalen Netzes kann beispielsweise mittels eines Backpropagation-Verfahrens durchgeführt werden. Dabei wird für das Netz eine möglichst zuverlässige Abbildung von gegebenen Eingabevektoren auf gegebene Ausgabevektoren angestrebt. Die Qualität der Abbildung wird durch eine Fehlerfunktion beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte.

Im trainierten Zustand enthalten die Verbindungsgewichte zwischen den Verarbeitungselementen Informationen bezüglich der Beziehung zwischen spektroskopischen Daten einer chemischen Verbindung und dem molekularen Deskriptor der chemischen Verbindung. Diese Informationen können verwendet werden, um molekulare Deskriptoren für neue chemische Verbindungen auf Basis deren spektroskopischer Daten vorherzusagen. Dabei bedeutet der Begriff "neu", dass die spektroskopischen Daten von einer chemischen Verbindung nicht bereits beim Trainieren des neuronalen Netzes verwendet worden sind. Das neuronale Netz ist also in der Lage, das angelernte "Wissen" auf andere chemische Verbindungen zu übertragen.

Eine Kreuzvalidierungsmethode kann verwendet werden, um die Trainingsdaten in Trainings- und Validierungsdatensätze aufzuteilen. Der Trainingsdatensatz wird beim Backpropagation-Training der Netzwerkgewichte verwendet. Der Validierungsdatensatz wird verwendet, um zu überprüfen, mit welcher Vorhersagegenauigkeit sich das trainierte Netzwerk auf unbekannte (neue) chemische Verbindungen (bzw. deren spektroskopische Daten) anwenden lässt.

Details zum Aufbau und zur Erzeugung von künstlichen neuronalen Netzen sind dem Stand der Technik zu entnehmen (siehe z.B.: R. Rojas: Neural Networks: A Systematic Introduction, Springer-Verlag 2013, ISBN: 9783642610684; D. Graupe: Principles Of Artificial Neural Networks: Basic Designs To Deep Learning, 4. Auflage, World Scientific Verlag, 2019, ISBN: 9789811201240).

Die spektroskopischen Daten, die zum Trainieren des ersten künstlichen neuronalen Netzes verwendet werden, können gemessene und/oder berechnete spektroskopische Daten sein. Spektroskopische Daten für eine Vielzahl an chemischen Verbindungen (Referenzverbindungen) sind z.B. in öffentlich zugänglichen Datenbanken zu finden (siehe z.B: https://pubchem.ncbi.nlm.nih.gov). SoftwareWerkzeuge zur Berechnung von NMR-Spektren sind kommerziell und frei verfügbar (siehe z.B.: https://www.nmrdb.org; http://www.cheminfo.org/; http://www.colby.edu/chemistry/NMR/scripts/C 13chemshift.html).

In einem weiteren Schritt wird der vom ersten künstlichen neuronalen Netz erzeugte molekulare Deskriptor dem zweiten neuronalen Netz zugeführt. Dabei können das erste neuronale Netz und das zweite neuronale Netz auch direkt miteinander verknüpft sein; die Ausgabeneuronen des ersten Netzes können direkt mit den Eingabeneuronen des zweiten neuronalen Netzes verbunden oder sogar mit diesen identisch sein. Dass in dieser Beschreibung dennoch von zwei neuronalen Netzen, dem ersten neuronalen Netz und dem zweiten neuronalen Netz die Rede ist, ist darin begründet, dass das erste neuronale Netz und das zweite neuronale Netz üblicherweise unabhängig voneinander konfiguriert (trainiert) werden. Sobald sie konfiguriert (trainiert) sind, können sie zu einem Netz zusammengefasst werden. Die vorliegende Erfindung soll also nicht so verstanden werden, dass das erste und das zweite neuronale Netz bei der Vorhersage von Strukturdaten auf Basis spektroskopischer Daten zwingend voneinander separiert sein müssen, sondern die Erfindung soll so verstanden werden, dass zur Vorhersage auch ein einheitliches Netz vorliegen kann, dass aus zwei unabhängig voneinander konfigurierten (trainierten) Teilnetzen zusammengesetzt ist.

Das zweite künstliche neuronale Netz ist ein Decoder eines Autoencoders. Ein Autoencoder besteht aus zwei künstlichen neuronalen Netzen (Teilnetzen), einem Encoder (Kodierer) und einem Decoder (Dekodierer). Das Ziel eines Autoencoders ist es üblicherweise, eine komprimierte Repräsentation (Encoding) für einen Satz Daten zu lernen und somit wesentliche Merkmale zu extrahieren. Dadurch kann er zur Dimensionsreduktion genutzt werden.

Im vorliegenden Fall überführt der Encoder Strukturdaten variabler Länge als Eingabesequenz in einen molekularen Deskriptor fester Größe. Der Decoder überführt den molekularen Deskriptor (zurück) in die Sequenz der Strukturdaten.

Der Autoencoder wird üblicherweise in einem unüberwachten Lernverfahren (*unsupervised learning*) trainiert, den Rekonstruktionsfehler auf Einzelzeichenebene für jede Eingabesequenz zu minimieren. Das Trainieren kann automatisiert erfolgen; d.h. der Autoencoder kann automatisiert anhand von Strukturdaten einer Vielzahl von chemischen Referenzverbindungen lernen, diese Strukturdaten variabler Länge in Codes fester Größe (molekulare Deskriptoren) zu transformieren und aus den molekularen Deskriptoren die Strukturdaten zu rekonstruieren.

Strukturdaten einer Vielzahl von chemischen Referenzverbindungen können Datenbanken wie beispielsweise PubChem (siehe z.B: https://pubchem.ncbi.nlm.nih.gov/; K. Sunghwan et al.: PubChem 2019 update: improved access to chemical data, Nucleic Acids Research, Volume 47, Issue D1, 2019, D1102-D1109, https://doi.org/10.1093/nar/gky1033) und/oder Zinc (siehe z.B.: http://zinc15.docking.org/; J. J. Irwin et al.: ZINC - a free database of commercially available compounds for virtual screening, J Chem Inf Model, 2005;45(1):177-182, doi:10.1021/ci049714) entnommen werden.

Weitere Details zu Autoencodern und deren Erzeugung sind beispielsweise zu finden in Q. V. Le: A Tutorial on Deep Learning Part 2: Autoencoders, Convolutional Neural Networks and Recurrent Neural Networks, 2015, https://cs.stanford.edu/~quocle/tutorial2.pdf; W. Meng: Relational Autoencoder for Feature Extraction, arXiv:1802.03145v1; WO2018046412A1).

Vorzugsweise basiert der verwendete Autoencoder auf der von Winter *et al.* veröffentlichten Architektur (siehe R. Winter et al.: Chem. Sci., 2019, 10, 1692-1701; Chem. Sci., 2020, 11, 10378-10389).

In einer bevorzugten Ausführungsform handelt es sich bei den molekularen Deskriptoren um kontinuierliche und datengetriebene molekulare Deskriptoren (engl.: *continuous and data-driven molecular descriptors*, Abk.: cddd), wie sie beispielsweise in den folgenden Publikationen beschrieben sind: T. Le, R. Winter, F, Noé, D.-A. Clevert: Neuraldecipher - reverse-engineering extendedconnectivity fingerprints (ECFPs) to their molecularstructures, Chem. Sci., 2020, 11, 10378; R. Winter, F. Montanari, F. Noé, D.-A. Clevert: Learning continous and data-driven molecular descriptors by translating equivalent chemical representations, Chem. Sci., 2019, 10, 1692).

In einem weiteren Schritt werden Strukturdaten der chemischen Verbindung als Ausgabe von dem zweiten künstlichen neuronalen Netz empfangen. Die Strukturdaten und/oder davon abgeleitete Informationen können in einem weiteren Schritt auf einem Bildschirm angezeigt, auf einem Drucker ausgegeben und/oder in einem Datenspeicher gespeichert werden.

Handelt es sich bei den Strukturdaten beispielsweise um einen Strukturcode für eine chemische Verbindung, so kann (neben oder anstelle der Strukturdaten) eine bildhafte Darstellung der chemischen Struktur der chemischen Verbindung auf einem Bildschirm angezeigt, auf einem Drucker ausgegeben und/oder in einem Datenspeicher gespeichert werden. Eine solche bildhafte Darstellung der chemischen Struktur kann beispielsweise eine Elektronenformel, Valenzstrichformel, Keilstrichformel und/oder eine Skelettformel sein (zur Darstellung chemischer Strukturen siehe z.B. J. K. Felixberger: Chemie für Einsteiger, Springer-Verlag 2017, ISBN: 978-3-662-52824-0, Kapitel 5.5 und S. Feil et al.: Faszinierende Chemie, Springer-Verlag 2017, ISBN: 978-3-662-49919-1, Seiten 32-33 und E. Benfenati et al.: Characterization of chemical structures, Chapter 3 of Quantitative Structure-Activity Relationships (QSAR) for Pesticide Regulatory Purposes, Elsevier-Verlag 2007, ISBN: 978-0-444-52710-3, Seiten 83 bis 109).

In einer Ausführungsform der vorliegenden Erfindung werden für die chemische Verbindung, deren Strukturdaten erfindungsgemäß aus spektroskopischen Daten vorhergesagt worden sind, spektroskopische Daten berechnet (z.B. ein ¹³C-NMR-Spektrum und/oder ein ¹H-NMR-Spektrum) und diese spektroskopischen Daten denjenigen spektroskopischen Daten gegenübergestellt, die zur Vorhersage verwendet worden sind. So kann das Ergebnis der Vorhersage unmittelbar überprüft und bewertet werden, indem die beiden spektroskopischen Datensätze miteinander verglichen werden. Bei einer korrekten Vorhersage stimmen die beiden spektroskopischen Datensätze überein. Bei einer fehlerhaften Vorhersage kann durch Identifikation der Abweichungen in den spektroskopischen Datensätzen ermittelt werden, mit welchen Strukturmerkmalen das erste künstliche neuronale Netz und/oder das zweite künstliche neuronale Netz noch Probleme haben. Anhand der Abweichungen können chemische Verbindungen für ein weiteres (aufbauendes) Training ermittelt und/oder ausgewählt und/oder erzeugt werden. Auch ein automatisiertes verstärkendes Lernen (engl.: *reinforced learning*) ist denkbar.

Die vorliegende Erfindung kann ganz oder teilweise mit einem Computersystem ausgeführt werden. Ein "Computersystem" ist ein System zur elektronischen Datenverarbeitung, das mittels programmierbarer Rechenvorschriften Daten verarbeitet. Ein solches System umfasst üblicherweise eine Steuer- und Recheneinheit, oftmals auch als "Computer" bezeichnet, diejenige Einheit, die einen Prozessor zur Durchführung logischer Operationen und einen Arbeitsspeicher zum Laden eines Computerprogramms umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Joystick, Laufwerke, Kamera, Mikrofon, Lautsprecher etc. Auch interne Anschlüsse und Erweiterungskarten gelten in der Computertechnik als Peripherie.

Computersysteme von heute werden häufig in Desktop-PCs, Portable PCs, Laptops, Notebooks, Netbooks, Tablet-PCs und so genannte Handhelds (z.B. Smartphone) unterteilt; alle diese Systeme können zur Ausführung der Erfindung genutzt werden.

Eingaben in das Computersystem (zum Bespiel zur Steuerung durch einen Nutzer) erfolgen über Eingabemittel wie beispielsweise eine Tastatur, eine Maus, ein Mikrofon, ein berührungsempfindliches Display und/oder dergleichen. Ausgaben erfolgen über eine oder mehrere Ausgabeeinheiten, die insbesondere ein Monitor (Bildschirm), ein Drucker und/oder ein Datenspeicher sein kann/können.

Die Erfindung wird nachstehend anhand von Zeichnungen näher erläutert, ohne die Erfindung auf die in den Zeichnungen gezeigten Merkmale oder Merkmalkombinationen beschränken zu wollen.

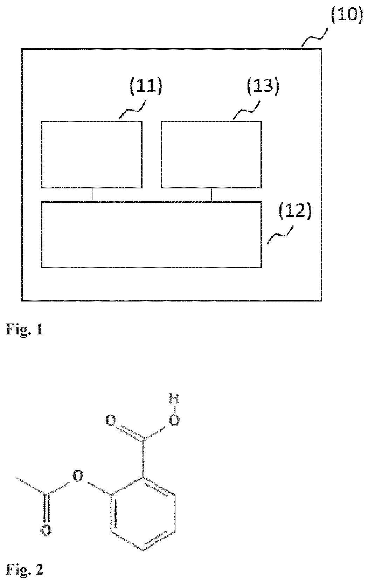

Fig. 1 zeigt schematisch und beispielhaft eine Ausführungsform des erfindungsgemäßen Computersystems. Das Computersystem (10) umfasst eine Empfangseinheit (11), eine Steuer- und Recheneinheit (12) und eine Ausgabeeinheit (13).

Das erfindungsgemäße Computersystem (10) ist konfiguriert, spektroskopische Daten chemischer Verbindungen zu empfangen, anhand der empfangenen spektroskopischen Daten Strukturdaten für die chemischen Verbindungen zu erzeugen und die Strukturdaten und/oder davon abgeleitete Informationen auszugeben und/oder zu speichern.

Die Steuer- und Recheneinheit (12) dient der Steuerung der Empfangseinheit (11) und der Ausgabeeinheit (13), der Koordinierung der Daten- und Signalflüsse zwischen den verschiedenen Einheiten, der Prozessierung von spektroskopischen Daten und weiteren Daten und der Erzeugung von Strukturdaten auf Basis der spektroskopischen Daten mittels eines ersten und eines zweiten künstlichen neuronalen Netzes. Das erste und das zweite künstliche neuronale Netz können beispielsweise in einem Arbeitsspeicher des Computersystems geladen sein, der ein Bestandteil der Steuer- und Recheneinheit (12) sein kann.

Die Empfangseinheit (11) dient zum Empfang von spektroskopischen Daten chemischer Verbindungen. Die spektroskopischen Daten können beispielsweise über ein Netzwerk (nicht in Fig. 1 dargestellt) von einem anderen Computersystem bereitgestellt/übermittelt und/oder aus einer Datenbank, die ein Bestandteil des erfindungsgemäßen Computersystems sein kann oder mit diesem über ein Netzwerk verbunden sein kann, ausgelesen werden.

Die Übermittlung von spektroskopischen Daten kann über eine Netzwerkverbindung oder eine direkte Verbindung erfolgen. Die Übermittlung von spektroskopischen Daten kann über eine Funkverbindung (WLAN, Bluetooth, Mobilfunk und/oder dergleichen) und/oder kabelgebunden erfolgen. Es ist denkbar, dass mehrere Empfangseinheiten vorhanden sind.

Von der Empfangseinheit werden die spektroskopischen Daten und ggf. weitere Daten an die Steuerund Recheneinheit (12) übermittelt. Die Steuer- und Recheneinheit (12) ist konfiguriert, anhand der empfangenen Daten Strukturdaten zu erzeugen.

Über die Ausgabeeinheit (13) können die Strukturdaten und/oder davon abgeleitete Informationen angezeigt werden (zum Beispiel auf einem Monitor), ausgegeben werden (z.B. über einen Drucker) oder in einem Datenspeicher gespeichert werden.

Es ist denkbar, dass mehrere Ausgabeeinheiten vorhanden sind. Ebenso können mehrere Empfangseinheiten und/oder Steuer- und/oder Recheneinheiten vorhanden sein.

Fig. 2 zeigt ein Beispiel für eine bildhafte Darstellung der chemischen Struktur einer chemischen Verbindung. Bei der Verbindung handelt es sich um Acetylsalicylsäure; der IUPAC-Name der Verbindung lautet 2-(Acetyloxy)benzoesäure (*engl.*: 2-acetoxybenzoic acid). Bei der bildhaft dargestellten Struktur handelt es sich um eine Skelettformel: die Kohlenstoffatome und die an Kohlenstoffatome gebundenen Wasserstoffatome sind nicht explizit dargestellt; lediglich die Sauerstoffatome (O) und das mit einem Sauerstoffatom verbundene Wasserstoffatom (H) sind durch ihre Elementsymbole (O, H) explizit dargestellt.

Der SMILES-Code für 2-(Acetyloxy)benzoesäure lautet: CC(=O)OC1=CC=CC=C1C(=O)O.

Der InChI-Code für 2-(Acetyloxy)benzoesäure lautet: 1S/C9H8O4/c1-6(10)13-8-5-3-2-4-7(8)9(11)12/h2-5H,1H3,(H,11,12).

Der SMILES-Code und der InChI-Code sind Beispiele für chemische Strukturcodes.

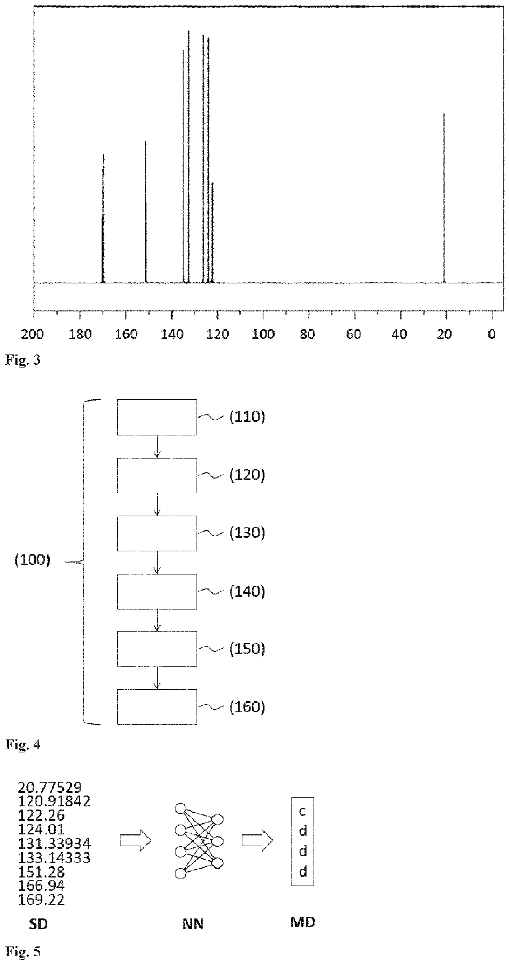

Fig. 3 zeigt beispielhaft ein ¹³C-NMR-Spektrum der in Figur 2 gezeigten chemischen Verbindung (2-(Acetyloxy)benzoesäure). Das Spektrum wurde in Deuterochloroform als Lösemittel (0,039g 2-(Acetyloxy)benzoesäure in 0,5 mL CDCL₃) bei 50,18 MHz. aufgenommen. Auf der Abszisse ist die chemische Verschiebung δ in ppm angegeben.

Ein Beispiel für eine Peak-Liste für ein berechnetes ¹³C-NMR-Spektrum (in diesem Fall für 2-(Acetyloxy)benzoesäure) ist:
δ (ppm)
20.77529
120.91842
122.26
124.01
131.33934
133.14333
151.28
166.94
169.22

Fig. 4 zeigt beispielhaft und schematisch eine Ausführungsform des erfindungsgemäßen Verfahrens in Form eines Ablaufdiagramms.

Das Verfahren (100) umfasst die Schritte:
(110) Empfangen von spektroskopischen Daten einer chemischen Verbindung,
(120) Zuführen der spektroskopischen Daten einem ersten künstlichen neuronalen Netz, wobei das erste künstliche neuronale Netz in einem überwachten Lernverfahren anhand von spektroskopischen Daten einer Vielzahl von chemischen Referenzverbindungen trainiert worden ist, auf Basis der spektroskopischen Daten der chemischen Referenzverbindungen molekulare Deskriptoren der chemischen Referenzverbindungen zu erzeugen,
(130) Empfangen eines molekularen Deskriptors der chemischen Verbindung von dem ersten künstlichen neuronalen Netz,
(140) Zuführen des empfangenen molekularen Deskriptors einem zweiten künstlichen neuronalen Netz, wobei das zweite künstliche neuronale Netz ein Decoder eines Autoencoders ist, wobei der Autoencoder in einem unüberwachten Lernverfahren anhand einer Vielzahl von chemischen Referenzverbindungen trainiert worden ist, Strukturdaten der chemischen Referenzverbindungen in molekulare Deskriptoren zu überführen und die molekularen Deskriptoren wieder in die Strukturdaten zu überführen,
(150) Empfangen von Strukturdaten der chemischen Verbindung von dem zweiten künstlichen neuronalen Netz,
(160) Ausgeben und/oder Speichern der Strukturdaten und/oder von Informationen, die von den Strukturdaten abgeleitet sind.

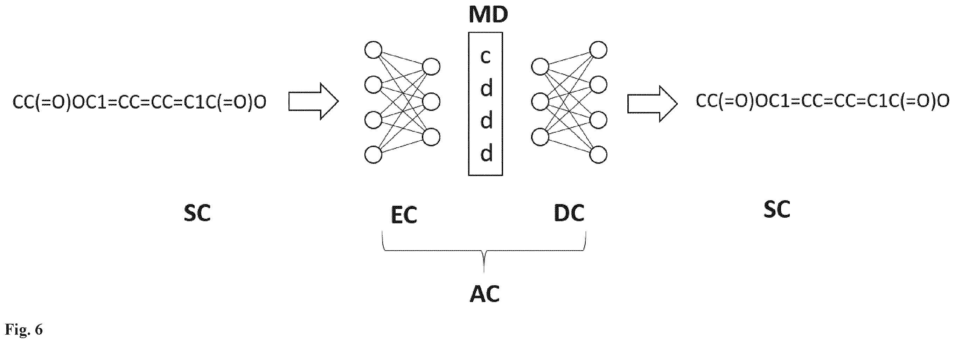

Fig. 5 zeigt beispielhaft und schematisch die Funktionsweise des ersten künstlichen neuronalen Netzes. Das erste neuronale Netz (NN) ist trainiert, aus spektroskopischen Daten (SD) als Eingabe einen molekularen Deskriptor (MD) zu erzeugen. Im vorliegenden Fall erzeugt es aus einer Peak-Liste eines ¹³C-NMR-Spektrums einen kontinuierlichen und datengetriebenen molekularen Deskriptor (cddd). Fig. 6 zeigt beispielhaft und schematisch die Funktionsweise eines Autoencoders. Der Autoencoder (AC) besteht aus einem Encoder (EC) und einem Decoder (DC). Der Autoencoder (AC) ist trainiert, aus Strukturdaten, im vorliegenden Fall aus einem chemischen Strukturcode einer chemischen Verbindung, einen molekularen Deskriptor (MD) zu erzeugen (Encoding) und aus dem molekularen Deskriptor (MD) den chemischen Strukturcode zu rekonstruieren (Decoding). Im vorliegenden Fall erzeugt der Encoder (EC) des Autoencoders (AC) aus dem SMILES-Code von 2-(Acetyloxy)benzoesäure (CC(=O)OC1=CC=CC=C1C(=O)O) einen kontinuierlichen und datengetriebenen molekularen Deskriptor (cddd) und der Decoder (DC) des Autoencoders (AC) aus dem kontinuierlichen und datengetriebenen molekularen Deskriptor (cddd) den SMILES-Codes von 2-(Acetyloxy)benzoesäure (CC(=O)OC1=CC=CC=C1C(=O)O).

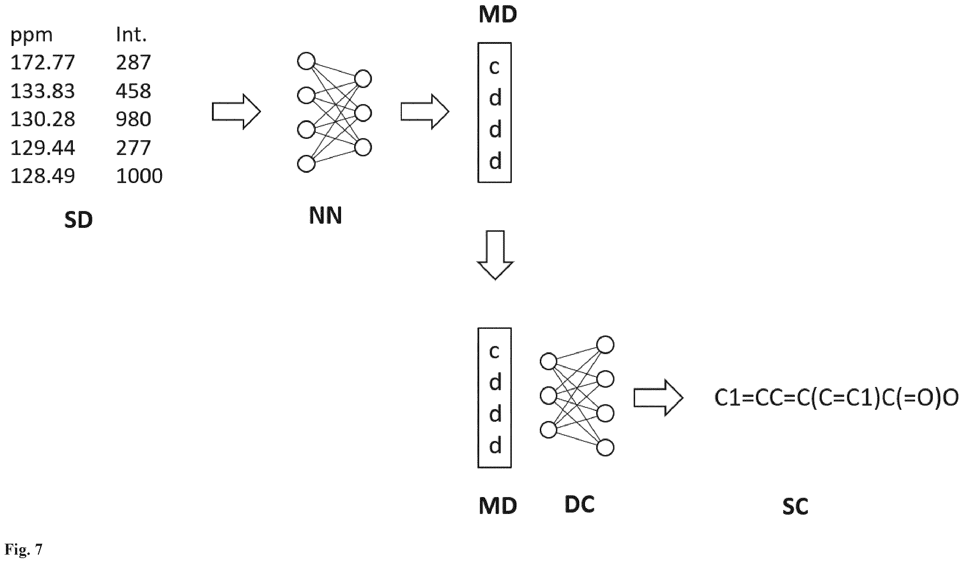

Fig. 7 zeigt beispielhaft und schematisch das Zusammenspiel des ersten und des zweiten künstlichen neuronalen Netzes bei der Erzeugung von Strukturdaten einer chemischen Struktur einer chemischen Verbindung aus spektroskopischen Daten der chemischen Verbindung. Bei dem ersten künstlichen neuronalen Netz (NN) handelt es sich um das in Fig. 5 gezeigte Netz. Bei dem zweiten künstlichen neuronalen Netz (DC) handelt es sich um den Decoder des in Fig. 6 gezeigten Autoencoders (AC). Das erste künstliche neuronale Netz (NN) empfängt spektroskopische Daten einer chemischen Verbindung (im vorliegenden Fall von Benzoesäure (engl.: *benzoic acid*))*.* Das erste künstliche neuronale Netz (NN) erzeugt aus den spektroskopischen Daten einen molekularen Deskriptor (MD), im vorliegenden Beispiel einen kontinuierlichen und datengetriebenen molekularen Deskriptor (cddd). Dieser kontinuierliche und datengetriebene molekulare Deskriptor (cddd) wird dem zweiten künstlichen neuronalen Netz (DC) zugeführt. Das zweite künstliche neuronale Netz (DC) erzeugt aus dem molekularen Deskriptor den SMILES-Code von Benzoesäure (C1=CC=C(C=C1)C(=O)O).

In der nachfolgenden Tabelle 1 sind eine Reihe von chemischen Strukturen chemischer Verbindungen in Form ihrer Skelettformel bildhaft dargestellt (mittlere Spalte). In der linken Spalte der Tabelle 1 ist für jede der chemischen Verbindungen eine Peakliste eines ¹³C-NMR-Spektren angegeben. In der rechten Spalte der Tabelle 1 sind die für die chemischen Verbindungen auf Basis der Peaklisten erfindungsgemäß vorhergesagten SMILES-Codes aufgeführt. Für die Vorhersage der SMILES-Codes wurde ein mehrschichtiges Feedforward-Neuronalnetz mit vollständig verbundenen Schichten als erstes neuronales Netz und der Decoder eines Autoencoders auf Basis der von Winter *et al.* beschriebenen Architektur als zweites neuronales Netz verwendet (Chem. Sci., 2019, 10, 1692-1701; Chem. Sci., 2020, 11, 10378-10389).

**Tabelle 1**

| | | |
|---|---|---|
| 14.3;0.0Q;9\|14.3;0.0Q;12\|24.8;0.0Q;5\|2 4.8;0.0Q;6\|61.4;0.0T;8\|61.4;0.0T;11\|123 .1;0.0S;1\|123.1;0.0S;4\|141.0;0.0D;0\|162 .1;0.0S;2\|162.1;0.0S;3\|165.7;0.0S;7\|165 .7;0.0S;10 | 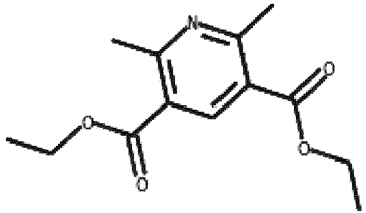 | CCOC(=O)c1cc(C(=O)OCC)c(C)nc1C |
| 15.1;0.0Q;10\|15.1;0.0Q;12\|60.9;0.0T;9\|6 0.9;0.0T;11\|84.4;0.08;6\|85.1;0.0S;7\|91. 8;0.0D;8\|121.9;0.0S;3\|128.2;0.0D;1\|128. 2;0.0D;5\|128.7;0.0D;0\|131.8;0.0D;2\|131. 8;0.0D;4 | 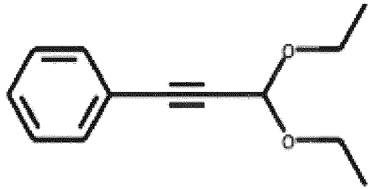 | CCOC(C#Cc1ccccc1)OCC |
| 14.6;0.0Q;7\|14.6;0.0Q;9\|62.2;0.0T;6\|62. 2;0.0T;8\|101.9;0.0S;0\|102.3;0.0D;5\|109. 8;0.0D;2\|113.3;0.0S;4\|121.2;0.0D;1\|123. 3;0.0D;3 | 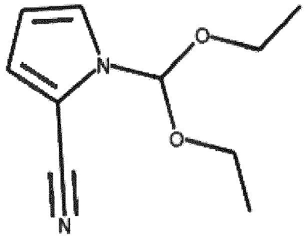 | CCOC(OCC)n1cccc1C#N |
| 52.32;0.0Q;9\|69.56;0.0T;6\|69.56;0.0T;7\| 106.91;0.0D;3\|107.87;0.0D;1\|107.87;0.0 D;5\|127.7;0.0D;11\|127.7;0.0D;15\|127.7; 0.0D;17\|127.7;0.0D;21\|127.92;0.0D;13\|1 27.92;0.0D;19\|128.46;0.0D;12\|128.46;0. 0D;14\|128.46;0.0D;18\|128.46;0.0D;20\|1 31.61;0.0S;0\|136.64;0.0S;10\|136.64;0.0 S;16\|159.51;0.0S;2\|159.51;0.0S;4\|165.8 3;0.0S;8 | 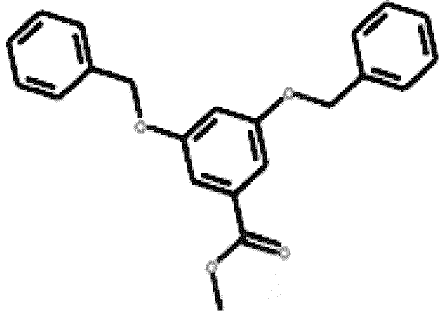 | COC(=O)c1cc(OCc2ccccc2)cc(OCc2ccccc2)c1 |
| 26.8;0.0Q;9\|124.4;0.0D;0\|124.4;0.0D;3\| 131.0;0.0S;4\|131.0;0.0S;5\|135.5;0.0D;1\| 135.5;0.0D;2\|165.3;0.0S;6\|165.3;0.0S;7\| 168.8;0.0S;8 | 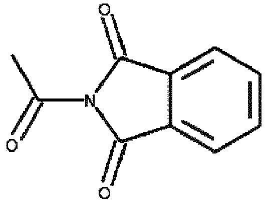 | CC(=O)N1C(=O)c2ccccc2C1=O |

## Patentansprüche

1. Computer-implementiertes Verfahren umfassend die Schritte:
- Empfangen von spektroskopischen Daten einer chemischen Verbindung,
- Zuführen der spektroskopischen Daten einem ersten künstlichen neuronalen Netz, wobei das erste künstliche neuronale Netz in einem überwachten Lernverfahren anhand von spektroskopischen Daten einer Vielzahl von chemischen Referenzverbindungen trainiert worden ist, auf Basis der spektroskopischen Daten der chemischen Referenzverbindungen molekulare Deskriptoren der chemischen Referenzverbindungen zu erzeugen,
- Empfangen eines molekularen Deskriptors der chemischen Verbindung von dem ersten künstlichen neuronalen Netz,
- Zuführen des empfangenen molekularen Deskriptors einem zweiten künstlichen neuronalen Netz, wobei das zweite künstliche neuronale Netz ein Decoder eines Autoencoders ist, wobei der Autoencoder in einem unüberwachten Lernverfahren anhand einer Vielzahl von chemischen Referenzverbindungen trainiert worden ist, Strukturdaten der chemischen Referenzverbindungen in molekulare Deskriptoren zu überführen und die molekularen Deskriptoren wieder in die Strukturdaten zu überführen,
- Empfangen von Strukturdaten der chemischen Verbindung von dem zweiten künstlichen neuronalen Netz,
- Ausgeben und/oder Speichern der Strukturdaten und/oder von Informationen, die von den Strukturdaten abgeleitet sind.

2. Verfahren gemäß Anspruch 1, wobei es sich bei den spektroskopischen Daten um Daten eines Kernresonanzspektrums oder mehrerer Kernresonanzspektren der chemischen Verbindung handelt.

3. Verfahren gemäß Anspruch 1 oder 2, wobei es sich bei den spektroskopischen Daten um eine Peak-Liste eines ¹³C-NMR-Spektrums und/oder eines ¹H-NMR-Spektrums handelt.

4. Verfahren gemäß einem der Ansprüche 1 bis 3, wobei es sich bei dem molekularen Deskriptor um einen n-dimensionalen Vektor handelt.

5. Verfahren gemäß einem der Ansprüche 1 bis 4, wobei es sich bei dem molekularen Deskriptor um einen kontinuierlichen und datengetriebenen molekularen Deskriptor handelt.

6. Verfahren gemäß einem der Ansprüche 1 bis 5, wobei es sich bei den Strukturdaten um einen chemischen Strukturcode handelt.

7. Verfahren gemäß einem der Ansprüche 1 bis 6, wobei es sich bei den Strukturdaten um einen SMILES-, InChI-, CML- oder WLN-Code handelt.

8. Verfahren gemäß einem der Ansprüche 1 bis 6, ferner umfassend die Schritte:
- Berechnen von spektroskopischen Daten aus den empfangenen Strukturdaten,
- Vergleichen der berechneten spektroskopischen Daten mit den empfangenen spektroskopischen Daten,
- Identifizieren der Abweichungen zwischen den berechneten spektroskopischen Daten und den empfangenen spektroskopischen Daten,
- Ausgeben und/oder Speichern der Abweichungen.

9. Computersystem umfassend
• eine Eingabeeinheit,
• eine Steuer- und Recheneinheit und
• eine Ausgabeeinheit
- wobei die Steuer- und Recheneinheit konfiguriert ist, die Eingabeeinheit zu veranlassen, spektroskopische Daten einer chemischen Verbindung zu empfangen,
- wobei die Steuer- und Recheneinheit konfiguriert ist, die spektroskopischen Daten einem ersten künstlichen neuronalen Netz zuzuführen, wobei das erste künstliche neuronale Netz in einem überwachten Lernverfahren anhand von spektroskopischen Daten einer Vielzahl von chemischen Referenzverbindungen trainiert worden ist, auf Basis der spektroskopischen Daten der chemischen Referenzverbindungen molekulare Deskriptoren der chemischen Referenzverbindungen zu erzeugen,
- wobei die Steuer- und Recheneinheit konfiguriert ist, von dem ersten künstlichen neuronalen Netz einen molekularen Deskriptor für die chemische Verbindung zu empfangen,
- wobei die Steuer- und Recheneinheit konfiguriert ist, den empfangenen molekularen Deskriptor einem zweiten künstlichen neuronalen Netz zuzuführen, wobei das zweite künstliche neuronale Netz ein Decoder eines Autoencoders ist, wobei der Autoencoder in einem unüberwachten Lernverfahren anhand einer Vielzahl von chemischen Referenzverbindungen trainiert worden ist, Strukturdaten der chemischen Referenzverbindungen in molekulare Deskriptoren zu überführen und die molekularen Deskriptoren wieder in die Strukturdaten zu überführen,
- wobei die Steuer- und Recheneinheit konfiguriert ist, von dem zweiten künstlichen neuronalen Netz Strukturdaten der chemischen Verbindung zu empfangen,
- wobei die Steuer- und Recheneinheit konfiguriert ist, die Ausgabeeinheit zu veranlassen, die empfangenen Strukturdaten und/oder Informationen, die von den Strukturdaten abgeleitet sind, auszugeben und/oder zu speichern.

10. Computerprogrammprodukt umfassend ein Computerprogramm, das in einen Arbeitsspeicher eines Computersystems geladen werden kann und dort das Computersystem dazu veranlasst, folgende Schritte ausführen:
- Empfangen von spektroskopischen Daten einer chemischen Verbindung,
- Zuführen der spektroskopischen Daten einem ersten künstlichen neuronalen Netz, wobei das erste künstliche neuronale Netz in einem überwachten Lernverfahren anhand von spektroskopischen Daten einer Vielzahl von chemischen Referenzverbindungen trainiert worden ist, auf Basis der spektroskopischen Daten der chemischen Referenzverbindungen molekulare Deskriptoren der chemischen Referenzverbindungen zu erzeugen,
- Empfangen eines molekularen Deskriptors der chemischen Verbindung von dem ersten künstlichen neuronalen Netz,
- Zuführen des empfangenen molekularen Deskriptors einem zweiten künstlichen neuronalen Netz, wobei das zweite künstliche neuronale Netz ein Decoder eines Autoencoders ist, wobei der Autoencoder in einem unüberwachten Lernverfahren anhand einer Vielzahl von chemischen Referenzverbindungen trainiert worden ist, Strukturdaten der chemischen Referenzverbindungen in molekulare Deskriptoren zu überführen und die molekularen Deskriptoren wieder in die Strukturdaten zu überführen,
- Empfangen von Strukturdaten der chemischen Verbindung von dem zweiten künstlichen neuronalen Netz,
- Ausgeben und/oder Speichern der Strukturdaten und/oder von Informationen, die von den Strukturdaten abgeleitet sind.

11. Computerprogrammprodukt gemäß Anspruch 10, wobei das Computerprogramm das Computersystem veranlasst, einen oder mehrere der Schritte der Ansprüche 1 bis 8 auszuführen.
